# EUROPEAN PATENT APPLICATION

(11) **EP 1 426 029 A1**
(43) Date of publication of application: **09.06.2004**
(21) Application number: 02743736.7
(22) Date of filing: 26.06.2002
(51) Int. Cl.: A61K 7/00, A61K 7/02, A61K 7/08, A61K 7/11, A61K 7/42, A61K 7/48, A61K 7/06

(54) **EMULSION COMPOSITIONS**

(30) Priority: 27.06.2001 JP 2001193970; 28.09.2001 JP 2001300068
(71) Applicant: Mandom Corporation, Osaka-shi, Osaka 540-8530 (JP)
(72) Inventor: KOBAYASHI, Aki, Osaka-shi, Osaka 540-8530 (JP); OKAMOTO, Hiroya, Osaka-shi, Osaka 540-8530 (JP); OKADA, Fumihiro, Osaka-shi, Osaka 540-8530 (JP); KANEHISA, Hidenori, Osaka-shi, Osaka 540-8530 (JP)
(74) Representative: Gemmell, Peter Alan, Dr.
(86) International application number: PCT/JP2002/006451
(87) International publication number: WO 2003/002074

(57) **Abstract**

Emulsion compositions which are excellent in long-term emulsion stability and feelings in use, and can be protected from putrefaction even without an antiseptic such as paraben or with a lowered amount of an antiseptic, if it is used. Namely, an emulsion composition characterized by comprising as the essential components a 1,2-alkanediol, a surfactant, an oily substance, and water; an emulsion composition as described above, characterized by containing at most 0.2 wt% of an antiseptic; and an emulsion composition as described above, characterized by being substantially free from an antiseptic.

## Description

### TECHNICAL FIELD

The present invention relates to emulsification compositions. More particularly, the present invention relates to the emulsification compositions which are excellent in emulsification stability and feelings in use. Further, the emulsification compositions are excellent in preservative stability even without antiseptic such as paraben and benzoic acid or with small amount of antiseptic.

### TECHNICAL BACKGROUND

In the past, in compositions of cosmetic, medicine and quasi medicine, formulation made from emulsion compositions contained surfactant solubilising oil content which is hardly soluble in water or aqueous solution is used. In these compositions, antiseptic such as paraben, benzoic acid and 2-hydroxybenzoic acid is used for preservative stability. Polyhydric alcohol such as Glycerin and 1,3-butylene glycol is compounded for improving feelings in use.

The conventional antiseptic is valnerable to pH status so that as a result, bad solution stability is confirmed. Antibacterial ability decreases conspicuously, when the antiseptic and composition such as surfactant are used at the same time. Large quantity of the antiseptic is required to maintain antiseptic effect.

Skin irritation may appear, when large quantity antiseptic is compounded. In these years, affection of paraben to a living body has been mattered. Further, the ministry stipulated that all ingredients compounded in cosmetic must be listed on labels, therefore, consumers gradually have concerned each compounded cosmetic component, and they prefer cosmetic without antiseptic such as paraben.

Polyhydric alcohol such as glycerin and 1,3-butylene glycol contributes to antiseptic ability, but compared with conventional antiseptic, its ability is ineffective. To obtain enough preservative effect from only said polyhydric alcohol, large amount composition such as 10∼20 weight% is required to compound. As a result, in the emulsion composition, emulsification balance of preparation is off-balance. Therefore, consistency decreases and separation occurs, and stabilized emulsion composition cannot be obtained. Further, such a high amount composition also has disadvantages that feeling is not excellent because of stickiness in and after use.

The present invention is performed to improve above-mentioned prior art, and its purpose is to provide emulsion compositions which are excellent in preservative stability even without antiseptic such as paraben and benzoic acid or with small amount of antiseptic.

### DESCRIPTION OF THE INVENTION

The invention described in claim 1 is emulsion compositions, wherein 1,2-alkanediol, surfactant, oily substance and water are contained as essential component.

The invention described in claim 2 is the emulsion compositions according to claim 1, wherein said 1,2-alkanediol is compound one and over described in a formula of R-CH(OH)-CH₂-OH (in the formula, R represents alkyl group that number of carbon is from 3 to 8).

The invention described in claim 3 is the emulsion compositions according to claim 1, wherein said 1,2-alkanediol is selected from one and over of 1,2-pentanediol, 1,2-hexanediol and 1,2-octanediol.

The invention described in claim 4 is the emulsion compositions according to any of claims 1 to 3, wherein said surfactant is selected from one and over of nonionic surfactant, anionic surfactant, cationic surfactant and ampholytic surfactant.

The invention described in claim 5 is the emulsion compositions according to any of claims 1 to 3, wherein said surfactant is the nonionic surfactant.

The invention described in claim 6 is the emulsion compositions according to any of claims 1 to 3, wherein said surfactant is the anionic surfactant.

The invention described in claim 7 is the emulsion compositions according to any of claims 1 to 3, wherein said surfactant is the cationic surfactant.

The invention described in claim 8 is the emulsion compositions according to any of claims 1 to 3, wherein said surfactant is the ampholytic surfactant.

The invention described in claim 9 is the emulsion compositions according to any of claims 1 to 8, wherein antiseptic is contained at most 0.2 weight %.

The invention described in claim 10 is the emulsion compositions according to any of claims 1 to 8, wherein the antiseptic is not contained substantially.

### EMBODIMEMT

Hereinafter, emulsion compositions of the present invention are described.

In the emulsion compositions, 1,2-alkanediol, surfactant, oily substance and water are compounded as essential components. 1,2-alkanediol is compounded because preservative effective or emulsion stability can be protected even without antiseptic or with small amount of the antiseptic. Antiseptic stability and emulsion stability are not decreased substantially.

Alkanes are aliphatic hydrocarbon and compounds of carbon and hydrogen. The alkanes have chain structure which are framed with carbon atoms. All bonds of the carbon atoms are single bond. Other atomic values of the carbon atoms bind to hydrogen atoms, and bond saturates.

1,2-alkanediols are composed by substituting hydrogen atom with hydroxyl group, and the kind of 1,2-alkanediols is not limited unless loosing above-mentioned effect. Following formula is shown as an example of 1,2-alkanediols.

R-CH(OH)-CH₂-OH

(in the formula, R represents alkyl group which number of carbon is from 3 to 8)

1,2-pentanediol, 1,2-hexanediol and 1,2-octanediol are preferably used in the view of preservative efficacy and obtainability. Further, it is more preferable to use the 1,2-hexanediol and 1,2-octanediol.

A composition amount of the 1,2-alkanediol is not limited unless loosing effect of the present invention. Composition rate from 0.01 to 10.0 weight% is preferable, and from 0.1 to 5.0 weight% is more preferable. Especially, in 1,2-pentanediol, composition amount from 1.0 to 5.0 weight% is more preferable. In 1,2-hexanediol, composition amount from 0.5 to 3.0 weight% is more preferable. In 1,2-octanediol, composition amount from 0.1 to 1.0 weight% is more preferable. If composition amount is below 0.01 weight%, antiseptic stability of the emulsion compositions is ineffective. If the composition amount is 10.0 and over weight%, the 1,2-alkanediol may smell and color the compositions, and either cases are not preferable.

As surfactant used in the present invention, surfactant used in cosmetics can be compounded, and any of nonionic surfactant, anionic surfactant, cationic surfactant and ampholytic surfactant can be used.

As the nonionic surfactant, for example, sorbitan fatty acid ester, glycerine fatty acid ester, castor oil, hardened castor oil, alkylene oxide addition product of these, polyglycerine fatty acid ester, polyoxialkylene alkyl ether, polyoxyalkylene fatty acid ester, polyoxialkylene alkylphenol, polyoxyethylene sorbitan fatty acid ester, polyoxyethylene alkylphenyl formaldehyde condensation product, polyoxyethylene sterol and its derivatives, polyoxyethylene lanoline and its derivatives, polyoxyethylene bees wax derivatives and sugar ester etc are exemplified.

As the anionic surfactant, for example, higher fatty acid soap, alkyl sulfate, alkyl phosphate, polyoxyethylene alkyl ester sulfate, polyoxyethylene alkyl phenyl ether sulfate, alkyl ether phosphate ether, alkyl ether carboxylate, acylmethyl taurate, N-acyl-N-methyl- β -alanine salt, N-acylglycine salt, N-acylglutamic acid, polyoxyethylene alkyl carboxylate, alkyl phenyl ether sulfonate, alkyl sulfosuccinate and its salt, N-acylsarcosine and its salt and polyoxyethylene coconut oil fatty acid monoethanol amide sulfate etc are exemplified.

As the cationic surfactant, for example, amine salt such as alkylamine salt, fatty acid amide amine salt, terciary amine salt-containing ester; alkyl quatermary ammonium salt such as monoalkyl type quatermary ammonium salt, dialkyl type quatermary ammonium salt, trialkyl type quatermary ammonium salt and benzalkonium type quatermary ammonium salt; cyclic quatermary ammonium salt such as alkylpyridinium salt etc and benzethonium chloride etc are exemplified.

As the ampholytic surfactant, for example, glycine type ampholytic surfactant such as alkyl glycine salt, carboxymethyl glycine salt, N-acyl aminoethyl-N-2-hydroxyethyl glycine salt etc; aminopropion acid type ampholytic surfactant such as alkylamino propionic acid salt, alkyl iminodipropionate salt etc; aminoacetic acid betaine type ampholytic surfactant such as alkyl dimethyl aminoacetic acid betaine and fatty acid amido propyl dimethyl aminoacetic acid betaine; sulfobetine type ampholytic surfactant such as alkyl hydroxy sulfobetine etc are exemplified.

The above-mentioned surfactants can be compounded separately or plurally. Compounded amount of the surfactant is not limited particularly, but it should be compounded from 0.01 to 20 weight%, more preferably from 0.1 to 10 weight%. In case of the compounded amount is under 0.01 weight%, an emulsification does not stabilize and compositions are apt to separate. In case of the compounded amount is over 20 weight%, a user feels sticky and uncomfortable in use, and it is also not preferable.

As compounded oily material, oily substances generally compounded in cosmetics can be used. These oily substances are not particularly limited, but for example, silicone oils such as methylpolysiloxane, methylphenylpolysiloxane, methylcyclopolysiloxane, octamethylcyclotetrasiloxane, octamethyl cyclopentasiloxane, decamethyl cyclopentasiloxane, methylhydrogenpolysiloxane; esters such as isopropyl myristate, 2-octyldodecyl myristate, cetyl 2-ethyl hexanoate, 2-ethylhexyl palmiate palminate, neopentiglycol di-2-ethylhexanoate, glycerol tri-2-ethylhexanoate, 2-octyldodecyl oleate, glycerol tri-isostearate, di-isostearyl malate and diglyceride 2-ethylhexanoate; higher alcohol such as cetyl alcohol, stearyl alcohol, isostearyl alcohol, 2-octyldodecanol and oleyl alcohol; higher fatty acids such as lauric acid, myristic acid, palmitic acid, stearic acid, isostearic acid and oleic acid; carbon hydrides such as liquid paraffin, paraffin, vaseline, ceresin, microcrystalline wax, squalene and squalane: wax such as carnauba wax, candelilla wax, jojoba oil, bees wax and lanoline; fat such as olive oil, camellia oil, macademia nut oil and avocado oil are exemplified.

The above-mentioned oily substances can be compounded separately or plurally. A compounded amount of the oily substances is not limited particularly, but it should be compounded from 0.1 to 90 weight%, more preferably from 1 to 60 weight%. When the compounded amount is under 0.1 weight%, an emulsification does not stabilize and compositions tend to separate, hence it is not preferable. When the compounded amount is over 90 weight%, feels in use is sticky and uncomfortable, and it is also not preferable.

As water used in the present invention, purified water, ion exchanged water and tap water are exemplified. It is preferable to use purified water and ion exchanged water since they are used in cosmetic.

Emulsion compositions of the present invention can be excluded or reduced the amount of preservative substantially by compounding 1,2-alkanediols. In case of antiseptic is used, antiseptic that is generally compounded in cosmetic is used at the same time.

As the used antiseptic, it is not limited particularly, but parabens such as methylparaben, ethylparaben and butylparaben; phenols such as isopropyl methyl phenol, cyclohexidine gluconate liquid, trichloro carbanilid, phenoxyethanol, carbolic and hexachlorophen etc; benzoic acid and its salt, photosensitizing dye No. 101, photosensitizing dye No. 201, photosensitizing dye No. 401, Hinokitiol and triclosan etc are exemplified.

Even if the preservative is used in the obtained emulsion compositions, the compounded amount of said preservative can be reduced. If the compounded amount of the preservative is less or equal to 0.2 weight%, antiseptic stability is still effective, and emulsion compositions without preservative can be obtained.

Further, in the emulsion compositions obtained from the present invention, thickening agents such as carboxyl vinyl polymer, medical agents such as glycyrrhizin, allantoin, vitamins, and extract of animals or plants, pearl agents, coloring agents, scrub agents, aroma chemicals, UV absorbents, antioxidants, chelating agents, pH adjusters, fine particles and diluting agents can be used optionally as long as a purpose of the present invention is kept.

The compounded amount of these materials is not limited particularly. and it should be adjusted optionally based on intended purpose of the emulsion compositions.

The emulsion compositions of the present invention can be used in sun protection products such as skin milk, essences, basic skin cares, sunscreens, makeup cosmetics such as foundations, eye liners, eyelash liners, eyecolors, cheekcolors and lip rouges, cleaning agents such as facial washes, body soaps and hair washes, hair dressing such as rinses, conditioners, hair creams, hair forms.

Hereinafter, the emulsion compositions of the present invention are explained based on examples, but the present invention is not limited by the examples. The unit of compounded amount is weight% unless other unit is used.

### (Preparation of samples)

Based on compositions described in table 1 to 4, each sample in the examples 1 to 25 and the comparative examples 1 to 8 were prepared by a common procedure and evaluated as follow.

### (Test example 1: evaluation of emulsion stability)

Each sample of the examples 1 to 25 and the comparative examples 1 to 8 were maintained constantly at the temperature of 5°C, 25°C and 40°C for one month. The emulsion stability of each sample after one month was evaluated based on following appraisal standard by visual observation. The result is shown in table 1 to 4.

### Appraisal standard

○ : Separation was not recognized.
Δ : A little separation was recognized.
× : Separation was recognized.

**Table 1**

| | | Examples | | | | | | | Comparative Examples | |
|---|---|---|---|---|---|---|---|---|---|---|
| | | 1 | 2 | 3 | 4 | 5 | 6 | 7 | 1 | 2 |
| squalane | | 7.00 | 7.00 | 7.00 | 7.00 | 7.00 | 7.00 | 7.00 | 7.00 | 7.00 |
| stearyl alcohol | | 4.00 | 4.00 | 4.00 | 4.00 | 4.00 | 4.00 | 4.00 | 4.00 | 4.00 |
| hydrogenated lanoline | | 1.50 | 1.50 | 1.50 | 1.50 | 1.50 | 1.50 | 1.50 | 1.50 | 1.50 |
| glyceryl monostearate | | 2.80 | 2.80 | 2.80 | 2.80 | 2.80 | 2.80 | 2.80 | 2.80 | 2.80 |
| polyoxyethylenecetylether | | 2.80 | 2.80 | 2.80 | 2.80 | 2.80 | 2.80 | 2.80 | 2.80 | 2.80 |
| pyrus cydonis seed extract | | 9.00 | 9.00 | 9.00 | 9.00 | 9.00 | 9.00 | 9.00 | 9.00 | 9.00 |
| perfume | | 0.15 | 0.15 | 0.15 | 0.15 | 0.15 | 0.15 | 0.15 | 0.15 | 0.15 |
| 1,2-pentanediol | | 1.00 | - | - | - | - | - | - | - | - |
| 1,2-hexanediol | | - | 0.50 | 1.00 | 1.00 | - | - | - | - | - |
| 1,2-octanediol | | - | - | - | - | 0.20 | 0.50 | 0.50 | - | - |
| 1,3-butyleneglycol | | - | - | - | - | - | - | - | 5.00 | 10.00 |
| methylparaben | | 0.15 | 0.15 | 0.15 | - | 0.15 | 0.15 | - | 0.15 | 0.15 |
| propylparaben | | 0.05 | 0.05 | 0.05 | - | 0.05 | 0.05 | - | 0.05 | 0.05 |
| purified water | | rest | rest | rest | rest | rest | rest | rest | rest | rest |
| Emulsion stability | 5°C | ○ | ○ | ○ | ○ | ○ | ○ | ○ | ○ | ○ |
| | 25°C | ○ | ○ | ○ | ○ | ○ | ○ | ○ | Δ | × |
| | 40°C | Δ | Δ | Δ | Δ | Δ | Δ | Δ | × | × |

**Table 2**

| | | Examples | | | | | | Comparative Examples | |
|---|---|---|---|---|---|---|---|---|---|
| | | 8 | 9 | 10 | 11 | 12 | 13 | 3 | 4 |
| liquid paraffin | | 5.0 | 5.0 | 5.0 | 5.0 | 5.0 | 5.0 | 5.0 | 5.0 |
| methylpolysiloxane | | 0.5 | 0.5 | 0.5 | 0.5 | 0.5 | 0.5 | 0.5 | 0.5 |
| behentrimoniumchloride | | 0.8 | 0.8 | 0.8 | 0.8 | 0.8 | 0.8 | 0.8 | 0.8 |
| dialkyldimethylammoniumchloride | | 1.0 | 1.0 | 1.0 | 1.0 | 1.0 | 1.0 | 1.0 | 1.0 |
| cetanol | | 1.5 | 1.5 | 1.5 | 1.5 | 1.5 | 1.5 | 1.5 | 1.5 |
| cationized cellulose | | 0.1 | 0.1 | 0.1 | 0.1 | 0.1 | 0.1 | 0.1 | 0.1 |
| 1,2-pentanediol | | 1.0 | 3.0 | - | - | - | - | - | - |
| 1,2-hexanediol | | - | - | 0.5 | 1.0 | - | - | - | - |
| 1,2-octanediol | | - | - | - | - | 0.2 | 0.5 | - | - |
| 1,3-butyleneglycol | | - | - | - | - | - | - | 5.0 | 10.0 |
| methylparaben | | 0.1 | - | 0.1 | - | 0.1 | - | 0.1 | 0.1 |
| purified water | | rest | rest | rest | rest | rest | rest | rest | rest |
| Emulsion stability | 5°C | ○ | ○ | ○ | ○ | ○ | ○ | ○ | ○ |
| | 25°C | ○ | ○ | ○ | ○ | ○ | ○ | Δ | × |
| | 40°C | Δ | Δ | ○ | Δ | ○ | Δ | × | × |

**Table 3**

| | | Examples | | | | | | Comparative Examples | |
|---|---|---|---|---|---|---|---|---|---|
| | | 14 | 15 | 16 | 17 | 18 | 19 | 5 | 6 |
| liquid paraffin | | 10.0 | 10.0 | 10.0 | 10.0 | 10.0 | 10.0 | 10.0 | 10.0 |
| wax | | 1.8 | 1.8 | 1.8 | 1.8 | 1.8 | 1.8 | 1.8 | 1.8 |
| sodium N-stearoyl-L-glutammate | | 1.2 | 1.2 | 1.2 | 1.2 | 1.2 | 1.2 | 1.2 | 1.2 |
| 1,2-pentanediol | | 1.0 | 3.0 | - | - | - | - | - | - |
| 1,2-hexanediol | | - | - | 0.5 | 1.0 | - | - | - | - |
| 1,2-octanediol | | - | - | - | - | 0.2 | 0.5 | - | - |
| 1,3-butyleneglycol | | - | - | - | - | - | - | 5.0 | 10.0 |
| methylparaben | | 0.1 | - | 0.1 | - | 0.1 | - | 0.1 | 0.1 |
| propylparaben | | 0.03 | - | 0.03 | - | 0.03 | - | 0.03 | 0.03 |
| purified water | | rest | rest | rest | rest | rest | rest | rest | rest |
| Emulsion stability | 5°C | ○ | ○ | ○ | ○ | ○ | ○ | ○ | ○ |
| | 25°C | ○ | ○ | ○ | ○ | ○ | ○ | Δ | Δ |
| | 40°C | Δ | Δ | ○ | Δ | ○ | Δ | × | × |

**Table 4**

| | | Examples | | | | | | Comparative Examples | |
|---|---|---|---|---|---|---|---|---|---|
| | | 20 | 21 | 22 | 23 | 24 | 25 | 7 | 8 |
| behenyl alcohol | | 2.0 | 2.0 | 2.0 | 2.0 | 2.0 | 2.0 | 2.0 | 2.0 |
| POE cetyl ether (20E. O.) | | 1.0 | 1.0 | 1.0 | 1.0 | 1.0 | 1.0 | 1.0 | 1.0 |
| POE cetyl ether (3E. O.) | | 1.2 | 1.2 | 1.2 | 1.2 | 1.2 | 1.2 | 1.2 | 1.2 |
| cocamidopropyl betaine, solution (30%) | | 20.0 | 20.0 | 20.0 | 20.0 | 20.0 | 20.0 | 20.0 | 20.0 |
| polyethylene (granule) | | 2.0 | 2.0 | 2.0 | 2.0 | 2.0 | 2.0 | 2.0 | 2.0 |
| 1,2-pentanediol | | 1.0 | 3.0 | - | - | - | - | - | - |
| 1,2-hexanediol | | - | - | 0.5 | 1.0 | - | - | - | - |
| 1,2-octanediol | | - | - | - | - | 0.2 | 0.5 | - | - |
| 1,3-butyleneglycol | | - | - | - | - | - | - | 5.0 | 10.0 |
| methylparaben | | 0.1 | - | 0.1 | - | 0.1 | - | 0.1 | 0.1 |
| purified water | | rest | rest | rest | rest | rest | rest | rest | rest |
| Emulsion stability | 5°C | ○ | ○ | ○ | ○ | ○ | ○ | ○ | ○ |
| | 25°C | ○ | ○ | ○ | ○ | ○ | ○ | Δ | Δ |
| | 40°C | Δ | Δ | ○ | Δ | ○ | Δ | × | × |

From the result of table 1 to 4, compared with emulsion compositions compounded 1,3-butylene glycol that is 1,3-alkanediol generally used in cosmetics, the emulsion compositions compounded 1,2-alkanediols perform excellent emulsion stability at 25°C. Further, in 40°C, it shows better emulsion stability than the comparative example.

### (Test example 2: evaluation of feelings)

Each sample of the examples 1 to 25 and the comparative examples 1 to 8 after evaluation of the emulsion stability at 25°C were used by five female panelists. Sensory evaluations of stickiness in use, moisture retention after use were evaluated based on following appraisal standard. The evaluations were employed the average amount of each panelist. The result is shown in table 5 to 8.

| Appraisal standard of stickiness | |
|---|---|
| Not sticky | 5 points |
| Slightly sticky, but not concerned | 4 points |
| Sticky is slightly concerned | 3 points |
| Sticky | 2 points |
| Very sticky | 1 point |

| Appraisal standard of moisture retention feelings | |
|---|---|
| Very moist | 5 points |
| Rather moist | 4 points |
| Moist | 3 points |
| Slightly moist | 2 points |
| Not moist | 1 point |

**Table 5**

| | | stickiness | | | | | | wetness | | | | | |
|---|---|---|---|---|---|---|---|---|---|---|---|---|---|
| | | female panelists | | | | | mean | female panelists | | | | | mean |
| | | A | B | C | D | E | | A | B | C | D | E | |
| Examples | 1 | 3 | 4 | 4 | 4 | 4 | 3.8 | 3 | 3 | 4 | 3 | 4 | 3.4 |
| | 2 | 4 | 3 | 4 | 5 | 4 | 4.0 | 3 | 3 | 3 | 4 | 3 | 3.2 |
| | 3 | 4 | 3 | 3 | 4 | 4 | 3.6 | 3 | 3 | 4 | 4 | 5 | 3.8 |
| | 4 | 4 | 3 | 2 | 4 | 4 | 3.4 | 3 | 4 | 4 | 4 | 5 | 4.0 |
| | 5 | 5 | 4 | 5 | 5 | 4 | 4.6 | 3 | 4 | 3 | 4 | 3 | 3.4 |
| | 6 | 4 | 4 | 5 | 5 | 4 | 4.4 | 3 | 4 | 3 | 3 | 4 | 3.4 |
| | 7 | 4 | 4 | 4 | 5 | 5 | 4.4 | 3 | 3 | 3 | 4 | 5 | 3.6 |
| Comparative Examples | 1 | 3 | 3 | 3 | 3 | 3 | 3.0 | 1 | 2 | 2 | 3 | 2 | 2.0 |
| | 2 | 2 | 2 | 3 | 2 | 2 | 2.2 | 2 | 3 | 3 | 3 | 3 | 2.8 |

**Table 6**

| | | stickiness | | | | | | wetness | | | | | |
|---|---|---|---|---|---|---|---|---|---|---|---|---|---|
| | | female panelists | | | | | mean | female panelists | | | | | mean |
| | | | B | C | D | E | | A | B | C | D | E | |
| Examples | 8 | 3 | 5 | 4 | 3 | 5 | 4.0 | 3 | 4 | 3 | 4 | 3 | 3.4 |
| | 9 | 4 | 3 | 4 | 3 | 4 | 3.6 | 4 | 5 | 4 | 5 | 5 | 4.6 |
| | 10 | 5 | 4 | 4 | 3 | 5 | 4.2 | 3 | 4 | 3 | 4 | 4 | 3.6 |
| | 11 | 3 | 4 | 3 | 4 | 5 | 3.8 | 4 | 4 | 4 | 5 | 4 | 4.2 |
| | 12 | 4 | 3 | 4 | 4 | 4 | 3.8 | 3 | 4 | 4 | 3 | 3 | 3.4 |
| | 13 | 4 | 3 | 4 | 4 | 4 | 3.8 | 4 | 5 | 4 | 3 | 4 | 4.0 |
| Comparative Examples | 3 | 3 | 3 | 3 | 3 | 3 | 3.0 | 3 | 3 | 2 | 2 | 3 | 2.6 |
| | 3 | 2 | 3 | 2 | 3 | 3 | 2.6 | 3 | 4 | 3 | 2 | 3 | 3.0 |

**Table 7**

| | | stickiness | | | | | | wetness | | | | | |
|---|---|---|---|---|---|---|---|---|---|---|---|---|---|
| | | female panelists | | | | | mean | female panelists | | | | | mean |
| | | A | B | C | D | E | | A | B | C | D | E | |
| Examples | 14 | 3 | 3 | 5 | 4 | 4 | 3.8 | 4 | 5 | 5 | 5 | 4 | 4.6 |
| | 15 | 3 | 4 | 4 | 3 | 4 | 3.6 | 5 | 5 | 4 | 5 | 5 | 4.8 |
| | 16 | 4 | 4 | 5 | 5 | 5 | 4.6 | 4 | 4 | 3 | 4 | 5 | 4.0 |
| | 17 | 4 | 4 | 4 | 4 | 5 | 4.2 | 5 | 4 | 4 | 5 | 5 | 4.6 |
| | 18 | 4 | 3 | 5 | 5 | 5 | 4.4 | 4 | 3 | 3 | 4 | 4 | 3.6 |
| | 19 | 4 | 4 | 5 | 5 | 5 | 4.6 | 4 | 4 | 3 | 4 | 5 | 4.0 |
| Comparative Examples | 5 | 3 | 3 | 3 | 3 | 4 | 3.2 | 3 | 4 | 2 | 3 | 4 | 3.2 |
| | 6 | 2 | 2 | 3 | 3 | 3 | 2.6 | 4 | 3 | 3 | 4 | 4 | 3.6 |

**Table 8**

| | | stickiness | | | | | | wetness | | | | | |
|---|---|---|---|---|---|---|---|---|---|---|---|---|---|
| | | female panelists | | | | | mean | female panelists | | | | | mean |
| | | A | B | C | D | E | | A | B | C | D | E | |
| Examples | 20 | 5 | 4 | 5 | 4 | 4 | 4.4 | 5 | 4 | 5 | 4 | 4 | 4.4 |
| | 21 | 4 | 3 | 3 | 4 | 4 | 3.6 | 5 | 5 | 5 | 4 | 5 | 4.8 |
| | 22 | 5 | 4 | 4 | 4 | 4 | 4.2 | 4 | 4 | 5 | 4 | 4 | 4.2 |
| | 23 | 5 | 4 | 4 | 3 | 4 | 4.0 | 5 | 5 | 5 | 4 | 4 | 4.6 |
| | 24 | 5 | 5 | 4 | 4 | 4 | 4.4 | 4 | 5 | 4 | 3 | 4 | 4.0 |
| | 25 | 4 | 4 | 4 | 4 | 3 | 3.8 | 4 | 5 | 5 | 4 | 4 | 4.4 |
| Comparative Examples | 7 | 3 | 3 | 3 | 3 | 3 | 3.0 | 3 | 4 | 3 | 2 | 3 | 3.0 |
| | 8 | 3 | 2 | 2 | 3 | 3 | 2.6 | 4 | 4 | 3 | 3 | 4 | 3.6 |

From table 5 to 8, it is figured out that the emulsion compositions compounded 1,2-alkanediols are not sticky but moist and further, feelings in use is good.

### (Test example 3: evaluation of preservative stability)

Preservative stability was evaluated with using each sample of the examples 1 to 7 and the comparative examples 1 and 2 by following operation.

In the test, as general bacteria, liquid of mix of Escherichia coli IF03972, Staphylococcus aureus IF013276, Bacteria subtilis IF012210 was used. Further, as yeast, Saccharomyces cerevisiae IF00234 was used. As mold, Aspergillus niger IF09455 was used. As mixture of general bacteria described above, incubation in advance was diluted 10⁸ cell/ml. As mixture of yeast described above, incubation in advance was diluted 10⁷ cell/ml. As mixture of mold described above, incubation in advance was diluted 10⁶ cell/ml. Numbers of cell were counted by colony count method.

In vials done dry heat sterilization, 20g of each sample of the examples 1 to 7 and the comparative examples 1 and 2 were put. The mixtures of bacteria was incubated at 35°C by inoculating the bacterial suspension 0.2 ml. The S. cerevisiae and A. niger were incubated at 25°C by inoculating the bacterial suspensions 0.2 ml. The numbers of living cells were calculated by the following methods. As to S. cerevisiae and mixture sample of bacteria; take out 1g of the mixture sample and of S. cerevisiae each on 1st day and 7th day after inoculating. Dilute with physiological saline and cultivate it in agar medium. After incubation for 48 hours, the number of living cells is determined. As to A. niger; take out 1g of A. niger on 1st day, and 14th day and 21st day after inoculating. Dilute with physiological saline and cultivate it in agar medium. After incubation for 48 hours, the number of living cells is determined. Regarding the A. niger, a measurement after recognition of complete annihilation was omitted. The result is shown in the table 9 to 11.

**Table 9**

| | | bacteria mixture (cells/g) | | |
|---|---|---|---|---|
| | | baseline value | 1 day after | 7 days after |
| Examples | 1 | 2.1×10⁶ | 3.4×10⁵ | 6.7×10³ |
| | 2 | | 9.8×10³ | 5.3×10² |
| | 3 | | 4.4×10³ | 1.0×10¹ |
| | 4 | | 6.3×10³ | 2.8×10¹ |
| | 5 | | 3.4×10³ | <10¹ |
| | 6 | | 2.0×10¹ | <10¹ |
| | 7 | | 8.1×10³ | 3.6×10¹ |
| Comparative Examples | 1 | | 8.9×10⁵ | 2.5×10⁵ |
| | 2 | | 3.6×10⁵ | 5.9×10⁵ |

**Table 10**

| | | S. cerevisiae (cells/g) | | |
|---|---|---|---|---|
| | | baseline value | 1 day after | 7 days after |
| Examples | 1 | 6.8×10⁵ | 4.9×10⁵ | 6.7×10³ |
| | 2 | | 2.3×10⁵ | <10¹ |
| | 3 | | 7.4×10³ | <10¹ |
| | 4 | | 4.6×10⁴ | 1.5×10² |
| | 5 | | 2.3×10³ | <10¹ |
| | 6 | | <10¹ | <10¹ |
| | 7 | | 6.2×10³ | 6.0×10¹ |
| Comparative Examples | 1 | | 8.9×10⁵ | 2.5×10⁵ |
| | 2 | | 3.6×10⁵ | 9.5×10⁴ |

**Table 11**

| | | A. niger (cells/g) | | | |
|---|---|---|---|---|---|
| | | baseline value | 1 day after | 7 days after | 21 days after |
| Examples | 1 | 1.7×10⁴ | 1.1×10⁴ | 2.7×10³ | 1.4×10² |
| | 2 | | 4.2×10³ | 5.7×10² | 8.0×10¹ |
| | 3 | | 1.8×10³ | 1.5×10² | 2.0×10¹ |
| | 4 | | 2.8×10³ | 3.3×10² | 1.0×10² |
| | 5 | | 6.2×10² | 3.0×10¹ | <10¹ |
| | 6 | | 2.2×10² | <10¹ | <10¹ |
| | 7 | | 7.3×10³ | 4.6×10² | 1.0×10² |
| Comparative Examples | 1 | | 3.3×10⁴ | 6.3×10⁴ | 8.4×10³ |
| | 2 | | 1.9×10⁴ | 9.9×10⁵ | 5.7×10³ |

From the result of the table 9 to 11, in emulsion compositions compounded 1,2-alkanediol, the number of cell decreases in time course even without antiseptic, and shows enough asepticus ability. However, in the comparative example used 1,3-butylene glycol, asepticus ability is inferior.

Hereinafter, formulation examples of the emulsification composition of the present invention are described. The unit of composition is weight%.

| Formulation example 1: emollient lotion (soft emulsion) | |
|---|---|
| vaseline | 2.0 |
| squalane | 5.0 |
| sorbitan sesquioleate | 0.8 |
| polyoxyethylene oleyl ether (20 E.O.) | 1.5 |
| 1,2-hexanediol | 1.5 |
| carboxyvinylpolymer | 0.16 |
| potassium hydroxide | 0.1 |
| methylparaben | 0.1 |
| perfume | adequate dose |
| UV absorbent | adequate dose |
| purified water | rest |
| Sum | 100.0 |

| Formulation example 2: emollient lotion (emulsion for rough skin) | |
|---|---|
| liquid paraffin | 15.0 |
| bees wax | 2.0 |
| lanolin | 1.5 |
| sorbitan sesquioleate | 2.5 |
| polyoxyethylene sorbitan monooleate (20E.O.) | 1.0 |
| 1,2-octanediol | 0.5 |
| propylene glycol | 3.0 |
| perfume | adequate dose |
| UV absorbent | adequate dose |
| purified water | rest |
| Sum | 100.0 |

| Formulation example 3: emollient cream | |
|---|---|
| stearyl alcohol | 5.0 |
| stearic acid | 2.0 |
| vaseline | 5.0 |
| squalane | 5.0 |
| dipropylene glycol | 4.0 |
| 1,2-pentanediol | 3.0 |
| propyleneglycol monostearate (20E.O.) | 2.5 |
| polyoxyethylene cetylether (20E.O.) | 3.0 |
| triethaol amine | 1.0 |
| methyl paraben | 0.1 |
| propyl paraben | 0.05 |
| perfume | adequate dose |
| UV absorbent | adequate dose |
| purified water | rest |
| Sum | 100.0 |

| Formulation example 4: massage cream | |
|---|---|
| paraffin | 5.0 |
| bees wax | 8.0 |
| vaseline | 8.0 |
| liquid paraffin | 30.0 |
| 1,2-octanediol | 0.5 |
| polyoxyethylene sorbitan monolaurate (20E.O.) | 2.0 |
| glyceryl monostearate | 1.6 |
| methyl paraben | 0.1 |
| propyl paraben | 0.1 |
| perfume | adequate dose |
| UV absorbent | adequate dose |
| purified water | rest |
| Sum | 100.0 |

| Formulation example 5: sunscreen emulsion | |
|---|---|
| stearic acid | 2.0 |
| liquid paraffin | 10.0 |
| cetanol | 1.0 |
| glyceryl monostearate, self-emulsifying | 1.0 |
| methylpolysiloxane | 2.0 |
| 1,2-octanediol | 0.5 |
| titanium oxide | 5.0 |
| triethaol amine | 0.8 |
| methyl paraben | 0.15 |
| perfume | adequate dose |
| purified water | rest |
| Sum | 100.0 |

| Formulation example 6: facial liquid foundation | |
|---|---|
| talc | 3.0 |
| titanium dioxide | 5.0 |
| bentonite | 0.5 |
| liquid paraffin | 8.0 |
| lanolin liquid | 2.0 |
| stearic acid | 2.0 |
| isohexadecyl alcohol | 7.0 |
| glyceryl monostearate | 2.0 |
| polyoxyethylene sorbitan monostearate | 0.9 |
| triethaol amine | 1.0 |
| 1,2-hexanediol | 2.0 |
| propylene glycol | 5.0 |
| methyl paraben | 0.1 |
| propyl paraben | 0.05 |
| color pigment | adequate dose |
| perfume | adequate dose |
| antioxidant | adequate dose |
| purified water | rest |
| Sum | 100.0 |

| Formulation example 7: mascara | |
|---|---|
| vinyl acetate emulsion | 20.0 |
| sodium carboxymethyl cellulose | 1.0 |
| 1,2-pentanediol | 3.0 |
| black iron oxide | 10.0 |
| kaolin | 2.0 |
| methyl paraben | 0.1 |
| purified water | rest |
| Sum | 100.0 |

| Formulation example 8: emulsifying-cream brushier | |
|---|---|
| white bees wax | 3.0 |
| stearic acid | 2.0 |
| polyoxyethylene sorbitan monostearate | 4.0 |
| glyceryl monostearate | 3.0 |
| cetanol | 1.0 |
| liquid paraffin | 20.0 |
| isopropyl palmitate | 3.0 |
| 1,2-octanediol | 0.3 |
| triethaol amine | 0.6 |
| titanium dioxide | 1.0 |
| talc | 6.0 |
| methyl paraben | 0.1 |
| propyl paraben | 0.1 |
| color pigment | adequate dose |
| purified water | rest |
| Sum | 100.0 |

| Formulation example 9: face washing form | |
|---|---|
| stearic acid | 10.0 |
| palmitic acid | 10.0 |
| myristic acid | 12.0 |
| lauric acid | 4.0 |
| oleyl alcohol | 1.5 |
| coconut oil | 2.0 |
| glycerin | 10.0 |
| polyethylene glycol 1500 | 4.0 |
| 1,2-pentanediol | 4.0 |
| potassium hydroxide | 6.0 |
| perfume | adequate dose |
| purified water | rest |
| Sum | 100.0 |

| Formulation example 10: hand cream | |
|---|---|
| liquid paraffin | 10.0 |
| vaseline | 5.0 |
| cetanol | 1.5 |
| sodium stearoyl glutamate | 1.0 |
| 1,2-octanediol | 0.5 |
| methyl paraben | 0.1 |
| propyl paraben | 0.05 |
| perfume | adequate dose |
| purified water | rest |
| Sum | 100.0 |

| Formulation example 11: cleansing cream | |
|---|---|
| paraffin | 5.0 |
| cetanol | 1.5 |
| vaseline | 20.0 |
| liquid paraffin | 25.0 |
| glyceryl monostearate | 3.0 |
| polyoxyethylene sorbitan laurate (20.E.0.) | 3.0 |
| 1,2-hexanediol | 2.0 |
| methyl paraben | 0.1 |
| perfume | adequate dose |
| purified water | rest |
| Sum | 100.0 |

| Formulation example 12: cleansing lotion | |
|---|---|
| stearic acid | 2.0 |
| cetanol | 1.5 |
| vaseline | 5.0 |
| liquid paraffin | 12.0 |
| polyoxyethylene oleyl ether (20 E.O.) | 1.8 |
| polyoxyethylene sorbitan laurate (5.E.0.) | 0.7 |
| 1,2-pentanediol | 3.0 |
| ethanol | 2.0 |
| methyl paraben | 0.1 |
| propyl paraben | 0.05 |
| color pigment | adequate dose |
| purified water | rest |
| Sum | 100.0 |

| Formulation example 13: conditioner hair lotion | |
|---|---|
| liquid paraffin | 3.0 |
| methylpolysiloxane | 2.5 |
| stearyl trimethyl ammonium chloride | 1.0 |
| dialkyl(12-18)dimethylammoniumchloride | 0.5 |
| 1,2-hexanediol | 1.0 |
| UV absorbent | adequate dose |
| perfume | adequate dose |
| purified water | rest |
| Sum | 100.0 |

| Formulation example 14: hair cream | |
|---|---|
| liquid paraffin | 12.0 |
| methylpolysiloxane | 5.0 |
| vaseline | 3.0 |
| cetanol | 0.5 |
| dicocoyl dimethyl ammonium chloride | 0.5 |
| lauryl trimethyl ammonium chloride | 2.0 |
| 1,2-hexanediol | 1.5 |
| UV absorbent | adequate dose |
| perfume | adequate dose |
| purified water | rest |
| Sum | 100.0 |

| Formulation example 15: hair wax | |
|---|---|
| liquid paraffin | 20.0 |
| methylpolysiloxane | 5.0 |
| cetanol | 3.0 |
| glycerin monostearate, self-emulsifying | 1.0 |
| polyoxyethylene sorbitan stearate (28E.O.) | 3.0 |
| 1,2-octanediol | 1.0 |
| glycerin | 3.0 |
| polyvinyl pyrrolidone | 2.0 |
| carboxy vinyl polymer | 0.35 |
| UV absorbent | adequate dose |
| perfume | adequate dose |
| purified water | rest |
| Sum | 100.0 |

| Formulation example 16: hair rinse | |
|---|---|
| methylpolysiloxane | 4.0 |
| liquid paraffin | 1.0 |
| cetanol | 3.0 |
| stearyl trimethyl ammonium chloride | 1.5 |
| 1,2-octanediol | 1.0 |
| glycerin | 3.0 |
| UV absorbent | adequate dose |
| perfume | adequate dose |
| purified water | rest |
| Sum | 100.0 |

### INDUSTRIAL APPLICATION

As mentioned above, since 1,2-alkanediol are compounded in the emulsion compositions, they are excellent in emulsion stability in time course and feelings in use without stickiness, and the present invention can be protected from putrefaction even without antiseptic such as paraben or with small amount of the antiseptic.

## Claims

1. Emulsion compositions, wherein 1,2-alkanediol, surfactant, oily substance and water are contained as essential component.

2. The emulsion compositions according to claim 1, wherein said 1,2-alkanediol is compound one and over described in a formula of R-CH(OH)-CH₂-OH (in the formula, R represents alkyl group that number of carbon is from 3 to 8).

3. The emulsion compositions according to claim 1, wherein said 1,2-alkanediol is selected from one and over of 1,2-pentanediol, 1,2-hexanediol and 1,2-octanediol.

4. The emulsion compositions according to any of claims 1 to 3, wherein said surfactant is selected from one and over of nonionic surfactant, anionic surfactant, cationic surfactant and ampholytic surfactant.

5. The emulsion compositions according to any of claims 1 to 3, wherein said surfactant is the nonionic surfactant.

6. The emulsion compositions according to any of claims 1 to 3, wherein said surfactant is the anionic surfactant.

7. The emulsion compositions according to any of claims 1 to 3, wherein said surfactant is the cationic surfactant.

8. The emulsion compositions according to any of claims 1 to 3. wherein said surfactant is the ampholytic surfactant.

9. The emulsion compositions according to any of claims 1 to 8, wherein antiseptic is contained at most 0.2 weight %.

10. The emulsion compositions according to any of claims 1 to 8, wherein the antiseptic is not contained substantially.
